# EUROPEAN PATENT APPLICATION

(11) **EP 4 478 075 A1**
(43) Date of publication of application: **18.12.2024**
(21) Application number: 24181826.9
(22) Date of filing: 12.06.2024
(51) Int. Cl.: G01S 7/00, G01S 7/41, G01S 13/02, G01S 13/56, G01S 13/87, G01S 13/88, G01S 13/89

(54) **SYSTEMS AND METHODS FOR MONITORING OCCUPANCY IN A ROOM**

(30) Priority: 16.06.2023 US 202363508571 P
(71) Applicant: Carrier Corporation, Palm Beach Gardens, FL 33418 (US)
(72) Inventor: GEHLAWAT, Prince, 500081 Hyderabad (IN); RAMOUTAR, Michael, Westford, MA 01886 (US); METTAIGARU, Rajani, 500081 Hyderabad (IN)
(74) Representative: Dehns

(57) **Abstract**

Embodiments of the disclosure describe systems and methods for monitoring occupancy in a room 120. The method comprises causing 302, via a detection unit 122, transmission of radar signals within the room 120 over a predefined period of time. The method further comprises receiving 304, via the detection unit, reflected signals corresponding to the transmitted radar signals, the reflected signals being indicative of information associated with the room 120. The method further comprises determining 306, based on the reflected signals, presence of one or more occupants within the room 120. The method further comprises, in response to determining presence of the one or more occupants within the room 120, determining 308 one or more of: an activity associated with the one or more occupants within the room 120, preferences associated with usage of the room 120 by the one or more occupants within the room 120, and a visualization map associated with the room.

## Description

This application claims the benefit of U.S. Provisional Patent Application No. 63/508,571 filed on June 16, 2023, which is incorporated by reference herein in its entirety.

The disclosure generally relates to detection systems, and more particularly relates to systems and methods to monitoring occupancy or presence of occupants in rooms.

Generally, establishments in hospitality industry offer rooms and accommodation spaces to users for a stay at the establishments. In a majority of cases, the rooms may remain unoccupied for a substantial duration as the users may not be present in their respective rooms. Further, existing techniques to detect occupancy of rooms are unable to provide results with high confidence. For instance, passive infrared (PIR) sensors, which are generally used to detect the presence of users in the rooms may have limitations with respect to detecting sleeping users, false motion detection, limited area coverage, etc. Blind spots may thus be present in the rooms and the PIR sensors may be unable to detect users in the blind spots.

Further, other sensors such as ultrasonic sensors and noise sensors may also not effective and accurate in detecting the presence of users in the rooms. Inaccurate detection of users in the room leads to higher energy costs, operational costs, and maintenance costs. For instance, maintenance staff may have to manually check whether rooms are occupied or not, such as prior to executing housekeeping tasks. Further, facilities within the room, such as lights, may continue to operate leading to energy consumption.

The hospitality industry is struggling with labour shortages, high energy costs, and high operational costs. Conventional techniques to detect occupancy in rooms are ineffective and do not facilitate tackling the above-discussed problems. Therefore, it would be advantageous to provide a solution that can overcome the above-discussed problems.

This summary is provided to introduce a selection of concepts, in a simplified format, that are further described in the detailed description of the disclosure. This summary is neither intended to identify key or essential inventive concepts of the disclosure and nor is it intended for determining the scope of the disclosure.

According to a first aspect of the invention there is provided a system for monitoring occupancy in a room as recited in claim 1. The system comprises a detection unit and a control unit communicatively connected with the detection unit. The control unit comprising one or more processors configured to cause, via the detection unit, transmission of radar signals within the room over a predefined period of time. The one or more processors are further configured to receive, via the detection unit, reflected signals corresponding to the transmitted radar signals, the reflected signals being indicative of information associated with the room. The one or more processors are further configured to determine, based on the reflected signals, presence of one or more occupants within the room. The one or more processors are further configured to, in response to determining presence of the one or more occupants within the room, determine one or more of: an activity associated with the one or more occupants within the room, preferences associated with usage of the room by the one or more occupants within the room, and a visualization map associated with the room.

Optionally, the detection unit comprises a first ultra-wide band (UWB) radar and a second UWB radar. The first UWB radar is configured to transmit radar signals within the room. The second UWB radar is configured to transmit the radar signals through wall structures within the room, thereby transmitting the radar signals throughout the room.

Optionally, the information associated with the room comprises one or more of occupant activity parameters related to activities of the one or more occupants within the room, occupant location parameters related to respective location of the one or more occupants within the room, and occupant vital parameters indicative of an identification of the one or more occupants within the room and vital sign information associated with the one or more occupants within the room.

Optionally, to determine presence of the one or more occupants, the one or more processors are further configured to compare one or more of frequency values, phase values, and amplitude values of the transmitted radar signals with one or more of frequency values, phase values, and amplitude values associated with the received reflected signals, respectively. The one or more processors are further configured to detect a change in the one or more of frequency values, phase values, and amplitude values of the reflected signals compared to the transmitted radar signals. The one or more processors are further configured to, in response to detecting the change, determine presence of the one or more occupants within the room.

Optionally, to determine the activity associated with the one or more occupants within the room, the one or more processors are configured to extract the occupant activity parameters based on the reflected signals. The one or more processors are further configured to determine an activity of the one or more occupants within the room based on the occupant activity parameters. The activity of the one or more occupants comprises one or more of sleeping, standing, sitting, exercising, and walking.

Optionally, the one or more processors are further configured to extract the occupant vital parameters based on the reflected signals. The one or more processors are further configured to determine, based on the occupant vital parameters, a health status associated with the one or more occupants within the room, wherein the health status comprises information related to sleep quality, sleep time, sleep stage, stress level, and health risks associated with the one or more occupants. The one or more processors are further configured to cause the health status to be displayed on a user interface associated with one or more of: the control unit, a user device of the one or more occupants, and a user device of one or more administrators associated with the room.

Optionally, the one or more processors are further configured to, upon determining the health status to comprise information related to health risks, transmit an alert notification to one or more of a user device of the one or more occupants, and the user device of one or more administrators associated with the room.

Optionally, to determine the preferences associated with the one or more occupants within the room, the one or more processors are configured to extract the occupant activity parameters and the occupant location parameters based on the reflected signals. The one or more processors are further configured to detect one or more surrounding parameters associated with the room, the one or more surrounding parameters being associated with a status of surroundings of the one or more occupants. The one or more processors are further configured to determine the preferences associated with a usage of the room based on the occupant activity parameters, the occupant location parameters, and the one or more surrounding parameters.

Optionally, the one or more processors are further configured to perform a control action based on the preferences associated with the one or more occupants. The control action comprises adjusting a status of surroundings of the occupant, wherein the status of surroundings comprises one or more of temperature status, light status, usage of one or more room areas, and stationary object status within the room.

Optionally, to determine the visualization map associated with the room, the one or more processors are configured to extract, based on reflected signals, the information associated with the room. The one or more processors are further configured to generate a virtual model of the room based on a pre-stored configuration of the room and the information associated with the room, the virtual model comprising a representation of the occupant within the room. The one or more processors are further configured to display the virtual model on one or more of a user interface associated with the one or more occupants and a user interface of an administrator associated with the room.

According to a second aspect of the invention there is provided a method for monitoring occupancy in a room as recited in claim 9. The method comprises causing, via a detection unit, transmission of radar signals within the room over a predefined period of time. The method further comprises receiving, via the detection unit, reflected signals corresponding to the transmitted radar signals, the reflected signals being indicative of information associated with the room. The method further comprises determining, based on the reflected signals, presence of one or more occupants within the room. The method further comprises, in response to determining presence of the one or more occupants within the room, determining one or more of an activity associated with the one or more occupants within the room, preferences associated with usage of the room by the one or more occupants within the room, and a visualization map associated with the room.

Optionally, the detection unit comprises a first ultra-wide band (UWB) radar and a second UWB radar, wherein the first UWB radar is configured to transmit radar signals within the room, and wherein the second UWB radar is configured to transmit the radar signals through wall structures within the room, thereby transmitting the radar signals throughout the room.

Optionally, the information associated with the room comprises one or more of occupant activity parameters related to activities of the one or more occupants within the room, occupant location parameters related to respective location of the one or more occupants within the room, and occupant vital parameters indicative of an identification of the one or more occupants within the room and vital sign information associated with the one or more occupants within the room.

Optionally, determining presence of the one or more occupants within the room comprises comparing one or more of frequency values, phase values, and amplitude values of the transmitted radar signals with one or more of frequency values, phase values, and amplitude values associated with the received reflected signals, respectively. The method further comprises detecting a change in one or more of the frequency values, phase values, and amplitude values of the reflected signals compared to the transmitted radar signals. The method further comprises in response to detecting the, determining presence of the one or more occupants within the room.

Optionally, determining the activity associated with the one or more occupants within the room comprises extracting the occupant activity parameters based on the reflected signals. The method further comprises determining an activity of the one or more occupants within the room based on the occupant activity parameters, wherein the activity of the one or more occupants comprises one or more of sleeping, standing, sitting, exercising, and walking.

Optionally, the method further comprises extracting the occupant vital parameters based on the reflected signals. The method further comprises determining, based on the occupant vital parameters, a health status associated with the one or more occupants within the room. The health status comprises information related to sleep quality, sleep time, sleep stage, stress level, and health risks associated with the one or more occupants. The method further comprises causing the health status to be displayed on a user interface associated with one or more of: the control unit, a user device of the one or more occupants, and a user device of one or more administrators associated with the room.

Optionally, the method further comprises upon determining the health status to comprise information related to health risks, transmitting an alert notification to one or more of a user device of the one or more occupants, and the user device of one or more administrators associated with the room.

Optionally, determining the preferences associated with the one or more occupants within the room comprises extracting the occupant activity parameters and the occupant location parameters based on the reflected signals. The method further comprises detecting one or more surrounding parameters associated with the room, the one or more surrounding parameters being associated with a status of surroundings of the one or more occupants. The method further comprises determining the preferences associated with a usage of the room based on the occupant activity parameters, the occupant location parameters, and the one or more surrounding parameters.

Optionally, the method further comprises performing a control action based on the preferences associated with the one or more occupants, wherein the control action comprises adjusting a status of surroundings of the occupant, wherein the status of surroundings comprises one or more of temperature status, light status, usage of one or more room areas, and stationary object status within the room.

Optionally, determining the visualization map associated with the room comprises extracting, based on the reflected signals, the information associated with the room. The method further comprises generating a virtual model of the room based on a pre-stored configuration of the room and the information associated with the room, the virtual model comprising a representation of the occupant within the room. The method further comprises displaying the virtual model on one or more of a user interface associated with the one or more occupants and a user interface of an administrator associated with the room.

The system of the first aspect of the invention may be configured for use with, or configured to perform, the method of the second aspect of the invention. The method of the second aspect of the invention may comprise using and/or providing the system of the first aspect of the invention.

To further clarify the advantages and features of the methods, systems, and apparatuses, a more particular description of the methods, systems, and apparatuses will be rendered by reference to specific embodiments thereof, which are illustrated in the appended drawings by way of example only. It is appreciated that these drawings depict only typical embodiments of the disclosure and are therefore not to be considered limiting of its scope. The disclosure will be described and explained with additional specificity and detail with the accompanying drawings.

These and other features, aspects, and advantages of the disclosure will become better understood when the following detailed description is read with reference to the accompanying drawings in which like characters represent like parts throughout the drawings, wherein:
**Figure 1** illustrates a system environment for monitoring occupancy in a room;
**Figure 2** illustrates a schematic block diagram of the system for monitoring occupancy in a room;
**Figure 3** illustrates a process flow chart depicting a method for monitoring occupancy in a room;
**Figure 4A** illustrates an additional process flow chart depicting a method for determining activity associated with one or more occupants within the room;
**Figure 4B** illustrates another additional process flow chart depicting a method for determining health status of one or more occupants within the room;
**Figure 4C** illustrate another additional process flow chart depicting a method for determining preferences associated with one or more occupants within the room; and
**Figure 5** illustrates a process flow depicting a method for processing signals from the detection unit to detect presence of one or more occupants in the room.

Further, skilled artisans will appreciate that elements in the drawings are illustrated for simplicity and may not have necessarily been drawn to scale. For example, the flow charts illustrate the method in terms of the most prominent steps involved to help to improve understanding of aspects of the disclosure. Furthermore, in terms of the construction of the device, one or more components of the device may have been represented in the drawings by conventional symbols, and the drawings may show only those specific details that are pertinent to understanding the embodiments of the disclosure so as not to obscure the drawings with details that will be readily apparent to those of ordinary skill in the art having the benefit of the description herein.

For the purpose of promoting an understanding of the principles of the disclosure, reference will now be made to the various embodiments and specific language will be used to describe the same. It will nevertheless be understood that no limitation of the scope of the disclosure is thereby intended, such alterations and further modifications in the illustrated system, and such further applications of the principles of the disclosure as illustrated therein being contemplated as would normally occur to one skilled in the art to which the disclosure relates.

It will be understood by those skilled in the art that the foregoing general description and the following detailed description are explanatory of the disclosure and are not intended to be restrictive thereof.

Reference throughout this specification to "an aspect", "another aspect" or similar language means that a particular feature, structure, or characteristic described in connection with the embodiment is included in at least one embodiment of the disclosure. Thus, appearances of the phrase "in an embodiment", "in another embodiment", "some embodiments", "one or more embodiments" and similar language throughout this specification may but do not necessarily, all refer to the same embodiment.

The terms "comprises", "comprising", or any other variations thereof, are intended to cover a non-exclusive inclusion, such that a process or method that comprises a list of steps does not include only those steps but may include other steps not expressly listed or inherent to such process or method. Similarly, one or more devices or sub-systems or elements or structures or components proceeded by "comprises... a" does not, without more constraints, preclude the existence of other devices or other sub-systems or other elements or other structures or other components or additional devices or additional sub-systems or additional elements or additional structures or additional components.

In addition to overcoming the challenges related to occupancy detection, the disclosure provides for a system that facilitates reduction in operational and maintenance costs. Further, health risks may be prevented as information related to vital signs of occupants in the room may be accurately collected. Furthermore, information related to preferences of the occupants and usage of room by the occupants may be utilized to provide better services to the occupants.

Embodiments of the disclosure will be described below in detail with reference to the accompanying drawings.

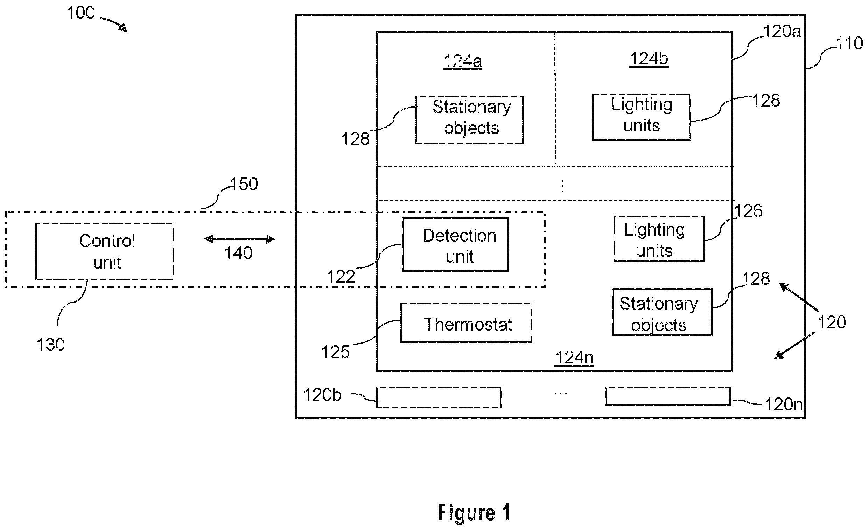

**Figure 1** illustrates a system environment 100 for monitoring occupancy in a room of a plurality of rooms.

The system environment 100 may include an indoor area/environment 110 comprising a plurality of rooms 120a-120n (interchangeably referred to as "120" hereinafter). In some embodiments, the indoor area 110 may be associated with a hotel, a lodge, a guest house, and/or any other establishment providing accommodation to users. A person skilled in the art would understand that the details explained with respect to one room 120 may be equally applicable for each of the plurality of rooms 120a-120n, without deviating from the scope of the disclosure.

In some embodiments, the room 120 may be associated with a detection unit 122. The detection unit 122 may be configured to transmit signals, such as radar signals, within the room 120. The detection unit 122 may further be configured to receive reflected signals corresponding to the transmitted signals. In some embodiments, the detection unit 122 may be an independent unit disposed at a suitable location within the room 120. For instance, the detection unit 122 may be disposed on a wall within the room 120. In some embodiments, the detection unit 122 may be integrated with a thermostat disposed within the room 120, and the functionalities of the detection unit 122 may be provided through the thermostat. In some embodiments, the detection unit 122 may be integrated into a digital signal processor (DSP) or a field-programmable gate array (FPGA) associated with the thermostat.

In some embodiments, the room 120 may be associated with different room areas 124a-124n within the room 120. The room areas 124a-124n may include, for instance, a bedroom, a study area (including a study table and chair), a bathroom, a living room, a balcony area, an area in vicinity of a minibar, and the like. In some embodiments, one or more of the room areas 124a-124n may be separated by wall structures. In some embodiments, the room 120 may be associated with a thermostat 125 configured to control the temperature within the room 120. For instance, the thermostat 125 may be configured to control the operation of a heating, ventilation, and air conditioning (HVAC) unit associated with the room 120 to control temperature and airflow within the room 120. In some embodiments, the room 120 may include one or more lighting units 126 configured to provide light within the room 120. In some embodiments, the room 120 may include one or more stationary objects 128 located at various positions within the room 120. The one or more stationary objects 128 may include, for instance, beds, fans, desks, chairs, and the like. In some embodiments, one or more of the thermostat 125 and the lighting units 126 may be communicatively coupled to the control unit 130, as discussed herein throughout the disclosure.

The detection unit 122 may be communicatively connected with a control unit 130 over a communication network 140, as discussed herein throughout the disclosure. The control unit 130 may be configured to monitor the occupancy in the room 120 based on the transmitted and reflected signals of the detection unit 122. The detection unit 122 and the control unit 130 may form a system 150 for monitoring the occupancy of the room 120. In some embodiments, the system 150 may additionally comprise one or more of the thermostat 125 and the lighting units 126.

In some embodiments, the control unit 130 may be a server-based device that is remote to the indoor area 110. In some embodiments, the control unit 130 may be a cloud-based device. In some embodiments, the control unit 130 may be integrated with the detection unit 122. In some embodiments, the control unit 130 may be integrated with the thermostat 125 within the room 120, and thus, the thermostat 125 may form part of the system 150 in such embodiments. In some embodiments, one or more components of the control unit 130 may be provided on a cloud while one or more components of the control unit 130 may be provided on a server or thermostat.

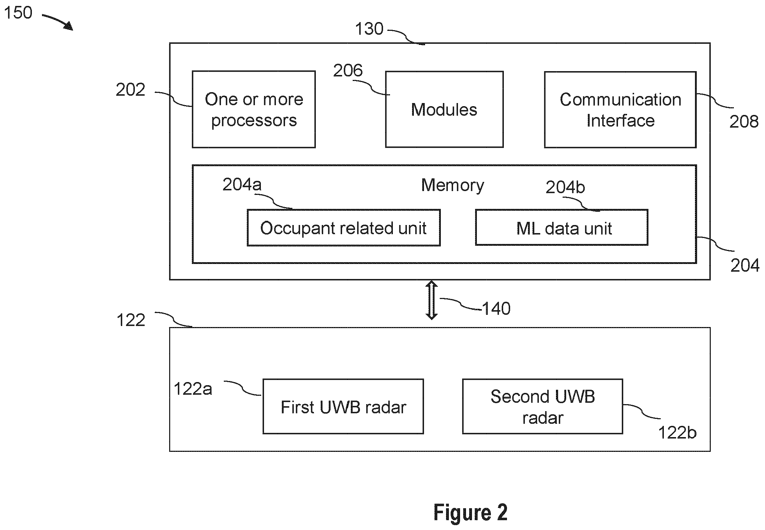

**Figure 2** illustrates a schematic block diagram of the system 150 for monitoring occupancy in a room of the plurality of rooms in the indoor area 110.

As shown in Figure 2, the system 150 comprises at least the control unit 130 and the detection unit 122. In some embodiments, the detection unit 122 may comprise a set of ultra-wide band (UWB) radars configured to transmit corresponding radar signals. The set of UWB radars may comprise a first UWB radar 122a and a second UWB radar 122b. In some embodiments, the first UWB radar 122a and the second UWB radar 122b may be arranged such that the first UWB radar 122a transmits radar signals inside the room 120 and the second UWB radar 122b transmits the radar signals through the wall structures within the room. Accordingly, the transmission of radar signals throughout the room 120 is achieved, in that, all areas within the room 120 are covered by the first and second UWB radars 122a, 122b.

In some embodiments, the first UWB radar 122a and the second UWB radar 122b may be configured on the basis of characteristics of the room 120, such as, room type, room size, etc., so as to achieve a 360 degrees detection coverage of the room 120. As an example, the first UWB radar 122a may be arranged to transmit radar signals in a bathroom area of the room 120, while the second UWB radar 122b may be arranged to transmit radar signals in the remaining areas of the room 120. In some embodiments, the first UWB radar 122a and the second UWB radar 122b may be positionally stitched back-to-back to form the set of UWB radars and achieve complete coverage of the room 120.

In some embodiments, the first UWB radar 122a and the second UWB radar 122b may each be one of a frequency-modulated continuous wave (FMCW) UWB radar, an impulse UWB radar, a M-sequence UWB radar, and the like. In some embodiments, each of the first UWB radar 122a and the second UWB radar 122b may be configured to operate within a frequency range of 3.1 to 10.6 GHz. It is appreciated that although an arrangement of dual UWB radars is depicted in Figure 2, the detection unit 122 may comprise, in some embodiments, more than two radars to achieve complete detection coverage of the room 120, and the details provided with respect to the first UWB radar 122a and the second UWB radar 122b are equally applicable for more than two UWB radars in the detection unit 122.

In one or more embodiments, the control unit 130 may comprise one or more processors 202, a memory 204, one or more modules 206, and a communication interface 208.

The one or more processors 202 may be configured to communicate with the memory 204 to store UWB radar-related data, such as data associated with transmitted radar signals and reflected signals, for monitoring occupancy of the room 120. In one or more embodiments, the one or more processors 202 may be one or more microprocessor(s) or microcontroller(s). The one or more processors 202 may include one or a plurality of processors, may include one or more general-purpose processors, such as a central processing unit (CPU), an application processor (AP), or the like, a graphics-only processing unit such as a graphics processing unit (GPU), a visual processing unit (VPU), and/or an Artificial intelligence (AI) dedicated processor such as a neural processing unit (NPU).

In some embodiments, the memory 204 may store data and instructions executable by the one or more processor(s) 202 to perform the method steps for monitoring occupancy of the room 120, as discussed herein throughout the disclosure. The memory 204 may further include, but is not limited to, a non-transitory computer-readable storage media such as various types of volatile and non-volatile storage media, including but not limited to, random access memory, read-only memory, programmable read-only memory, electrically programmable read-only memory, electrically erasable read-only memory, flash memory, magnetic tape or disk, optical media, and the like. Further, the non-transitory computer-readable storage media of the memory 204 may include executable instructions in the form of the modules 206 and a database to store data. The modules 206 may include a set of instructions that may be executed to cause the one or more processors 202 to perform any one or more of the methods for monitoring occupancy of the room 120, as disclosed herein throughout the disclosure. Specifically, the one or more modules 206 may be configured to perform the steps of the disclosure using the data stored in the database of the memory 204 for monitoring occupancy of the room 120. In another embodiment, the modules 206 may be one or more hardware units that may be outside the memory 204. In one embodiment, the memory 204 may communicate via a bus within the one or more processor(s) 202.

In one or more embodiments, the communication interface 208 may include a transmitter and a receiver, and further, may be configured to communicate with the detection unit 122, via the communication network 140. In some embodiments, the communication interface 208 may be configured to communicate with one or more of the thermostat 125 and the lighting units 126, via the communication network 140. The communication via the communication network 140 may be based on a wireless communication protocol. The communication interface 208 may be configured for communicating internally between internal hardware components and with external devices, e.g., the detection unit 122, via one or more networks (e.g., radio technology). The communication interface 208 may include an electronic circuit specific to a standard that may enable wireless communication. In some embodiments, the communication network may be implemented using Wi-Fi, long range (LoRa), Global System for Mobile Communication (GSM), narrow-band internet of things (NBIOT), and the like.

Referring to Figures 1 and 2, the one or more processors 202 may be configured to cause, via the detection unit 122, transmission of radar signals within the room. The radar signals may be transmitted over a predefined period of time. As described above, the radar signals may be transmitted by the first UWB radar 122a and the second UWB radar 122b so as to cover each area of the room 120. The transmitted radar signals, when spread within the room 120, may be reflected by one or more stationary objects 128 within the room 120. Further, in case of presence of one or more occupants within the room 120, the transmitted radar signals are also reflected by the body of the one or more occupants.

The one or more processors 202 may be configured to receive, via the detection unit 122, reflected signals corresponding to the transmitted radar signals. The one or more processors 202 may further be configured to determine presence of one or more occupants within the room 120 based on the reflected signals. In some embodiments, the one or more processors 202 may be configured to determine presence of the one or more occupants based on one or more of phase values, amplitude values, and frequency values of the transmitted radar signals and the reflected signals.

In some embodiments, the one or more processors 202 may be configured to compare one or more of frequency values, phase values, and amplitude values of the transmitted radar signals with one or more of frequency values, phase values, and amplitude values associated with the reflected signals, respectively. When the radar signals from the detection unit 122 transmit through the body of the one or more occupants, the corresponding reflected signals may contain fluctuations in one or more of phase values, amplitude values, and frequency values. As an example, the fluctuations may be caused by various body parts (arms, limbs, torso, etc.) of the one or more occupants. As another example, the fluctuations may be caused by chest surfaces during inhaling and exhaling by the one or more occupants.

In some embodiments, the one or more processors 202 may be configured to detect a change in the one or more of frequency values, phase values, and amplitude values of the reflected signals compared to the one or more of frequency values, phase values, and amplitude values of the transmitted radar signals. A change in the one or more of frequency values, phase values, and amplitude values of the reflected signals may be indicative of presence of one or more occupants within the room 120. In some embodiments, the one or more processors 202 may detect the change based on one or more signal processing and clutter removal algorithms. In some embodiments, signal to noise ratio associated with the reflected signals may be improved by suppressing non-stationary clutter using singular value decomposition (SVD). In some embodiments, linear trend subtraction (LTS) technique may be used to remove stationary clutter and linear trends associated with the reflected signals. In some embodiments, complex signal demodulation (CSD) may be used to suppress harmonics associated with the reflected signals. In some embodiments, the one or more processors 202 may filter the reflected signals to eliminate stationary objects prior to detecting the change. In some embodiments, the one or more processors 202 may be configured to determine the presence of one or more occupants within the room 120 in response to detecting the change in the one or more of frequency values, phase values, and amplitude values of the reflected signals as compared to the one or more of frequency values, phase values, and amplitude values of the transmitted radar signals.

As the transmitted radar signals may be reflected based on the presence of occupants within the room 120, the reflected signals may be indicative of information associated with the room 120. Further, as the transmitted radar signals may be reflected based on the presence of objects within the room 120, the reflected signals may be indicative of one or more surrounding parameters associated with a status of the surroundings (objects) within the room 120.

In some embodiments, the information associated with the room 120 comprises one or more of occupant activity parameters, occupant location parameters, and occupant vital parameters. The occupant activity parameters may relate to activities of the one or more occupants within the room 120, such as, standing, walking, sitting, sleeping, and the like. The occupant location parameters may relate to the respective location of the one or more occupants within the room 120. The occupant vital parameters may be indicative of identification of the one or more occupants and/or vital sign information associated with the one or more occupants within the room 120.

The one or more processors 202 may be configured to determine, in response to determining presence of the one or more occupants within the room 120, additional data based on one or more of the occupant activity parameters, the occupant location parameters, and the occupant vital parameters. The additional data may comprise one or more of an activity associated with the one or more occupants within the room 120, preferences associated with usage of the room by the one or more occupants within the room 120, and/or a visualization map associated with the room 120.

In some embodiments, the one or more processors 202 may be configured to determine the activity associated with the one or more occupants based on the occupant activity parameters. The one or more processors 202 may be configured to extract the occupant activity parameters based on the reflected signals which are indicative of the information associated with the room 120. Based on the extracted occupant activity parameters, the one or more processors 202 may be configured to determine the activity of the one or more occupants within the room 120. In some embodiments, the activity of the one or more occupants includes one or more of sleeping, standing, sitting, exercising, walking, and the like.

In some embodiments, the one or more processors 202 may determine the activity of the one or more occupants using a first machine learning (ML) model. In some embodiments, the first ML model may be stored in the memory 204, such as, in an ML data unit 204b of the memory 204. In some embodiments, the first ML model may be based on deep learning algorithms. In some embodiments, the first ML model may include wavelet neural networks. For instance, based on the first ML model, the one or more processors 202 may determine the activity of an occupant of the one or more occupants to be sleeping. The first ML model may process the occupant activity parameters to determine the heights of chest, arms, and limbs from the ground, whether the position of the chest and arms are lower as compared to a standing position, whether the length of the arms is perpendicular or parallel to the ground, and the like. Based on the processing of the first ML model, an output may be generated indicative of the determined activity of the occupant. In some embodiments, the occupant activity parameters, and the data associated with the determined activity of the one or more occupants, may be stored in an occupant-related unit 204a within the memory 204.

In some embodiments, the one or more processors 202 may be configured to determine a health status of the one or more occupants based on the occupant vital parameters. The one or more processors 202 may be configured to extract the occupant vital parameters based on the reflected signals which are indicative of the information associated with the room 120. Based on the extracted occupant vital parameters, the one or more processors 202 may be configured to determine the health status of the one or more occupants within the room 120.

In some embodiments, the occupant vital information may include information associated with the cardiovascular and respiratory systems of the one or more occupants. In some embodiments, the occupant vital information may include information associated with the heartbeat and breathing of the one or more occupants. In some embodiments, the health status of the one or more occupants may include information related to sleep quality, sleep time, sleep stage, stress level, and health risks associated with the one or more occupants. In some embodiments, the one or more processors 202 may determine the health status of the one or more occupants using a second machine learning (ML) model. In some embodiments, the second ML model may be stored in the memory 204, such as, in the ML data unit 204b of the memory 204. In some embodiments, the second ML model may include wavelet neural networks. In some embodiments, the second ML model and the first ML model may form a part of a common ML model configured to detect activity of the one or more occupants as well as configured to determine health status of the one or more occupants.

In some embodiments, a training system may be used to train the first, second, and/or the common ML model, in accordance with one or more embodiments of the present disclosure. In some embodiments, the training system may use unsupervised learning algorithms to train the models. In some embodiments, using unsupervised learning algorithms may be beneficial because it may allow for discovering hidden trends and patterns, or extracting data features from the input data that would have been difficult to obtain if other techniques were used. It is to be understood that the machine learning models described herein are examples. However, other techniques, are also contemplated by the present disclosure. As such, any computer implemented techniques, or machine learning techniques for determining activity of the occupants, health status of the occupants, and/or for determining occupancy are contemplated by the present disclosure. For example, supervised learning, semi-supervised, unsupervised learning, reinforcement learning, and/or other machine learning techniques are contemplated by the present disclosure. In some embodiments, the machine learning models may include decision trees, support vector machines, regression analysis, Bayesian networks, random forest learning, dimensionality reduction algorithms, boosting algorithms, artificial neural networks (e.g., fully connected neural networks, deep convolutional neural networks, or recurrent neural networks), deep learning, and/or other machine learning models.

In some embodiments, the one or more processors 202 may be configured to cause the determined health status to be displayed on one or more user interfaces. In some embodiments, the one or more user interfaces comprise user interface associated with the control unit 130, such as, in a non-limiting example, the thermostat 125 within the room 120. In some embodiments, the one or more user interfaces comprise user interface associated with the one or more occupants, such as, in a non-limiting example, user interfaces of hand-held devices of the one or more occupants. In some embodiments, the one or more user interfaces comprise user interface associated with the one or more administrators associated with the room 120 and/or the indoor area 110. For instance, the user interface associated with the one or more administrators may include a user interface of a common device, such as a device at a reception area associated with the indoor area 110, and/or a user interface associated with handheld devices associated with the one or more administrators.

In some embodiments, the one or more processors 202 may be configured to generate and transmit alert notifications upon determining the health status to comprise information related to health risks. In some embodiments, the one or more processors 202 may be configured to transmit an alert notification to a user device (such as a handheld device) of the one or more occupants. In some embodiments, the one or more processors 202 may be configured to transmit an alert notification to a user device (such as a handheld device) of the one or more administrators associated with the room 120 and/or the indoor area 110. Accordingly, the occupants and/or the administrators may be made aware of possible health risks so that necessary precautions and/or remedies may be taken in promptly.

In some embodiments, the one or more processors 202 may process the occupant location parameters and/or the occupant vital parameters, in addition to the occupant activity parameters, in order to determine the activity associated with the one or more occupants. In one example, the one or more processors 202 may determine that for an occupant of the one or more occupants, the occupant is in a still position and the arms and limbs are parallel to the ground, thereby indicating that the occupant may be sleeping. In another example, the one or more processors 202 may determine that location of the occupant is changing at a particular rate and the arms and legs are moving, thereby indicating that the occupant may be walking. In other examples, the one or more processors 202 may determine that the occupant may be standing when the location of the occupant is constant and the arms and legs are perpendicular to the ground, the occupant may be exercising based on respiration rates and heart-beat related information of the occupant, the occupant may be sitting based on the position of the upper body with respect to the legs, the occupant may have fallen based on a rate of change in position of the occupant from a standing position to a sitting position.

In some embodiments, the one or more processors 202 may be configured to determine the preferences associated with the one or more occupants within the room 120. In some embodiments, the preferences associated with the one or more occupants may include preferences of the one or more occupants in relation to the usage of the room 120. For example, the one or more occupants may prefer to spend the majority time in area 124a of the plurality of areas 124a-124n within the room 120. As another example, the one or more occupants may prefer to use a particular object (such as a particular chair) of the objects 128 within the room 120 as compared to the remaining objects 128.

In some embodiments, the one or more processors 202 may be configured to extract the occupant activity parameters and the occupant location parameters based on the reflected signals which are indicative of the information associated with the room 120. In some embodiments, the one or more processors 202 may extract the occupant location parameters based on reception angles of the reflected signals, i.e., angles at which the reflected signals are received at the detection unit 122. In some embodiments, the one or more processors 202 may extract the occupant location parameters based on one or more of localization and tracking techniques. In some embodiments, discrete short-time Fourier transform (DSTF) technique may be used to calculate distance between the one or more occupants and the detection unit 122.

In some embodiments, the one or more processors 202 may be configured to detect one or more surrounding parameters associated with the room 120. The one or more surrounding parameters may be associated with a status of surrounding of the one or more occupants. In some embodiments, the surroundings of the one or more occupants may include the thermostat 125, the lighting units 126, and/or the stationary objects 128 within the room 120. Based on the extracted occupant activity parameters, occupant location parameters, and the one or more surrounding parameters, the one or more processors 202 may be configured to determine the preferences associated with the one or more occupants, in particular, preferences associated with the usage of the room 120 and the surroundings by the one or more occupants within the room 120.

In some embodiments, the one or more processors are configured to perform a control action based on the preferences associated with the one or more occupants. The control action may comprise adjusting one or more of the status of surroundings of the occupant. In some embodiments, the status of surroundings comprises one or more of temperature status, light status, usage of one or more room areas 124a-124n, and status of stationary objects 128 within the room 120. As an example, the control action may comprise sending an activation or deactivation signal to the lighting units 126 in one or more of the room areas 124a-124n, thereby adjusting the light status within the room 120. As another example, the control action may comprise adjusting the operation of the thermostat 125, thereby adjusting the temperature status within the room 120. In some embodiments, the control action may comprise sending an adjustment notification to one or more administrators to facilitate adjusting the status of the stationary objects 128 and/or adjusting usage of one or more room areas 124a-124n within the room 120.

In some embodiments, the one or more processors 202 may be configured to determine the visualization map associated with the room 120 based on the information associated with the room 120. The one or more processors 202 may be configured to extract the information associated with the room 120 based on the reflected signals, the information associated with the room 120 comprising at least the occupant activity parameters and the occupant location parameters.

In some embodiments, a configuration of the room 120 may be pre-stored in the memory 204, the configuration comprising information related to a type, shape, size, and structure of the room 120, information related to the plurality of areas 124a-124n within the room 120, placement of stationary objects 128 within the room 120, and the like. The one or more processors 202 may be configured to generate a virtual model of the room 120 based on the pre-stored configuration of the room 120 as well as based on the information associated with the room 120. In some embodiments, the virtual model may comprise a representation of the occupant within the room 120. In some embodiments, the virtual model may comprise representations of the stationary objects 128 within the room 120. In some embodiments, the one or more processors 202 may be configured to display the virtual model of the room 120 on one of more of the user interface associated with the one or more occupants, such as on the user device of the one or more occupants, and/or the user interface associated with one or more administrators, such as on the user device of the one or more administrators. In some embodiments, the one or more occupants and/or the one or more administrators may view the virtual model through a software application installed in the respective user devices and/or through a web-based application accessible via the respective user devices.

In some embodiments, the one or more processors 202 may be configured to integrate the determined preferences associated with the usage of the room 120 with the virtual model of the room 120. Accordingly, the visualization map may comprise the virtual model of the room 120 along with information regarding usage of the room 120 by the one or more occupants.

In some embodiments, the information associated with the room 120 may be stored in the memory 204, for instance, in the occupant related unit 204a of the memory 204. In some embodiments, the information associated with the memory may be stored in a pre-determined format such that the information may be retrieved by the one or more processors 202. In some embodiments, the information may be stored in a format comprising {`occupant detection status,' 'occupant identifier', 'occupant coordinates', 'occupant activity'}. In some embodiments, the information may be stored in a format comprising {`number of occupants', 'occupant identifier', 'occupant coordinates', 'occupant occupied area', 'occupant activity', `light status', 'temperature status', 'blinds status', 'sleep duration', 'sleep stage', 'timestamp'}.

The occupant detection status may be '0x01' which indicates the presence of occupants or '0x00' which indicates an absence of occupants. The occupant identifier may be an identifier specific to an occupant within the room 120 such that in case of multiple occupants in the room 120, each of the multiple occupants may be identified. For instance, a first occupant within the room 120 may be identified as 'A' and a second occupant within the room 120 may be identified as 'B'.

The occupant coordinates may be indicative of location of the one or more occupants within the room 120. For instance, coordinates (10,01) may indicate that one of the occupants is present within the room 120 at a particular location referred to by coordinates (10,01). The occupant activity may be indicative of an activity of the one or more occupants within the room 120. For instance, the occupant activity '1' may be indicative of a sleeping activity, occupant activity `2' may be indicative of a walking towards door activity, occupant activity '3' may be indicative of a standing activity, occupant activity `4' may be indicative of a workout activity, occupant activity '5' may be indicative of a sitting activity, occupant activity `6' may be indicative of a walking away from door activity, occupant activity '7' may be indicative of a fallen down activity, etc.

The number of occupants in the format may be indicative of a total number of occupants in the room and may be represented as an integer value. The occupant occupied area may be indicative of a specific area of the plurality of areas 124a-124n within the room 120 where the occupant may be present. For instance, '01' may indicate that occupant is at the minibar while '02' may indicate that occupant is in the bathroom. The light status may indicate the operation of one or more of the lighting units 126 within the room 120. For instance, `L1' may indicate that lighting unit 126 labelled L1 is active. The temperature status may indicate temperature of the room 120 which may be represented in numerical form. The blinds status may indicate whether the blinds in the room 120 are open or closed, in that, '0' may indicate that blinds are open while '1' may indicate that blinds are closed. The sleep duration may indicate the time duration for which the occupant was sleeping, which may be indicated in a numerical form. The sleep stage may indicate a stage of sleep of the occupant, such as, '1' may indicate REM sleep cycle, `2' may indicate core sleep cycle, `3' may indicate deep sleep cycle, and the like. The timestamp may indicate a time when the information is received, such as, in 'hh:mm' or 'dd:mm:yy' form.

As an example, information in format {0x01, A, (10,01), 1} may indicate that an occupant identified as 'A' is present in the room 120 at a location (10,01) and is sleeping. In another example, information in format {1,1,(06,05),0,2,L1,L2,20,0,0,0,18:00,01-01-23} may indicate that at 6pm, the occupant is at the minibar while information in format {1,1, (06,03),3,1,0,22, O,0,0,20:30,01-01-23} may indicate that the occupant is on bed, sleeping, with lights turned off, and temperature set at 22 degrees. Accordingly, the information associated with the room 120 may be used for obtaining data with respect to preferences of the occupant regarding usage of the room 120, and additionally, automatically control operation of various components within the room such as lighting units 126 and thermostat 125. Moreover, data may be obtained for multiple occupants within the room and the components may be controlled taking into account the preferences of each of the multiple occupants.

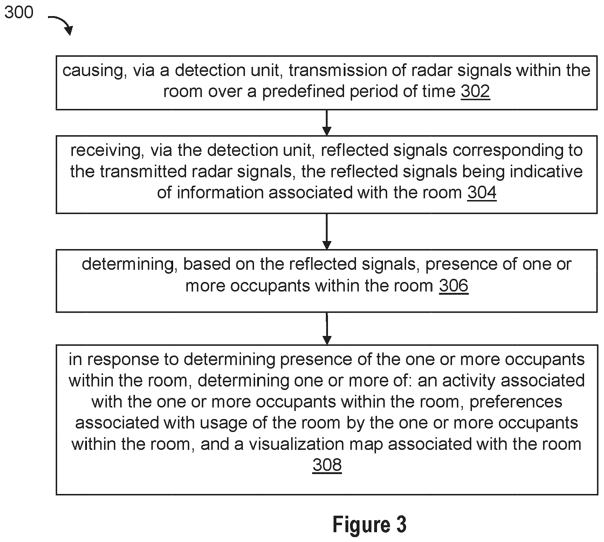

**Figure 3** illustrates a process flow depicting a method 300 for monitoring occupancy in a room, such as the room 120. The method 300 may be described with reference to room 120, and a skilled person will appreciate that the method may be performed for each of the plurality of rooms 120a-120n in the indoor area 110.

At step 302, the method 300 comprises causing, via the detection unit 122, transmission of radar signals within the room 120 over a predefined period of time. In some embodiments, the detection unit 122 comprises the first UWB radar 122a and the second UWB radar 122b.

At step 304, the method 300 comprises receiving, via the detection unit 122, reflected signals corresponding to the transmitted radar signals. The reflected signals may be indicative of information associated with the room. In some embodiments, the information associated with the room comprises one or more of occupant activity parameters, occupant location parameters, and occupant vital parameters.

At step 306, the method 300 comprises determining, based on the reflected signals, presence of one or more occupants within the room 120.

At step 308, the method 300 comprises, in response to determining presence of the one or more occupants within the room 120, determining one or more of an activity associated with the one or more occupants within the room 120, preferences associated with usage of the room by the one or more occupants within the room 120, and a visualization map associated with the room 120.

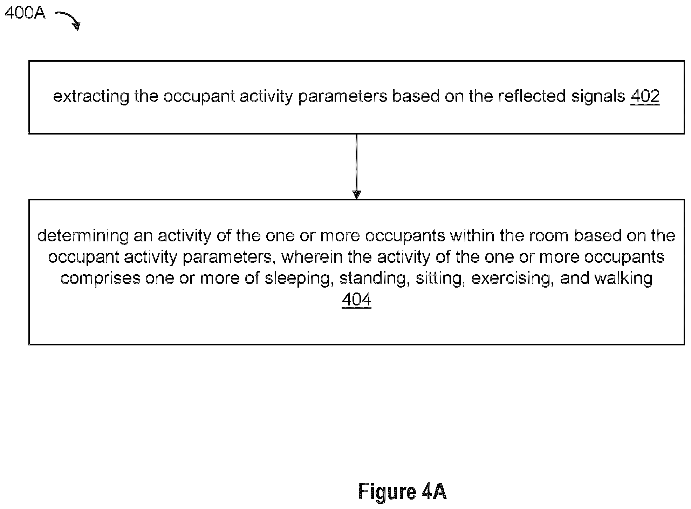

In some embodiments, the method 300 may comprise additional steps to determine the activity associated with the one or more occupants within the room 120. **Figure 4A** illustrates an additional process flow depicting a method 400A that, at additional step 402, comprises extracting the occupant activity parameters based on the reflected signals. In some embodiments, signal processing and clutter removal techniques may be used to extract the occupant activity parameters.

At additional step 404, the method 400A comprises determining an activity of the one or more occupants within the room based on the occupant activity parameters. The activity of the one or more occupants may comprise one or more of sleeping, standing, sitting, exercising, and walking.

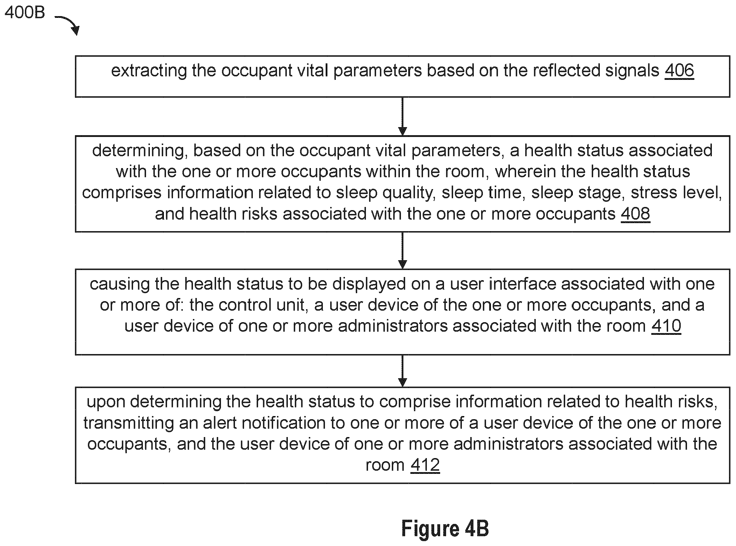

In some embodiments, the method 300 may comprise additional steps to determine a health status of the one or more occupants in the room 120. **Figure 4B** illustrates an additional process flow depicting a method 400B that, at additional step 406, comprises extracting the occupant vital parameters based on the reflected signals.

At additional step 408, the method 400B comprises determining, based on the occupant vital parameters, a health status associated with the one or more occupants within the room 120. The health status may comprise information related to sleep quality, sleep time, sleep stage, stress level, and health risks associated with the one or more occupants. The health risks may include, for instance, whether the occupant has fallen in the room.

At additional step 410, the method 400B comprises causing the health status to be displayed on a user interface associated with one or more of the control unit 130, a user device of the one or more occupants, and a user device of one or more administrators associated with the room 120.

At additional step 412, the method 400B comprises, upon determining the health status to comprise information related to health risks, transmitting an alert notification to one or more of a user device of the one or more occupants, and the user device of one or more administrators associated with the room 120.

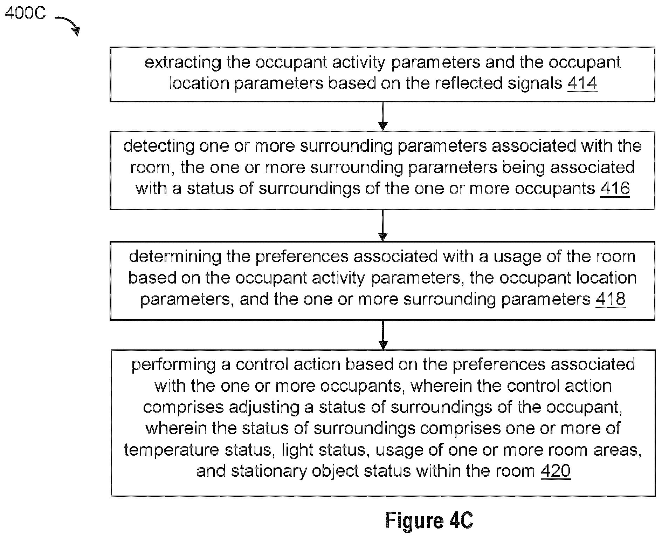

In some embodiments, the method 300 may comprise additional steps to determine the preferences associated with the one or more occupants within the room 120. The preferences may comprise preferences related to usage of the room 120 by the one or more occupants. **Figure 4C** illustrates an additional process flow depicting a method 400C that, at additional step 414, comprises extracting the occupant activity parameters and the occupant location parameters based on the reflected signals.

At additional step 416, the method 400C comprises detecting one or more surrounding parameters associated with the room. The one or more surrounding parameters may be associated with the status of surroundings of the one or more occupants.

At additional step 418, the method 400C comprises determining the preferences associated with the usage of the room based on the occupant activity parameters, the occupant location parameters, and the one or more surrounding parameters.

At additional step 420, the method 400C comprises performing a control action based on the preferences associated with the one or more occupants. The control action may comprise adjusting the status of the surroundings of the occupant. The status of surroundings may comprise one or more of temperature status, light status, usage of one or more room areas, and stationary object status within the room.

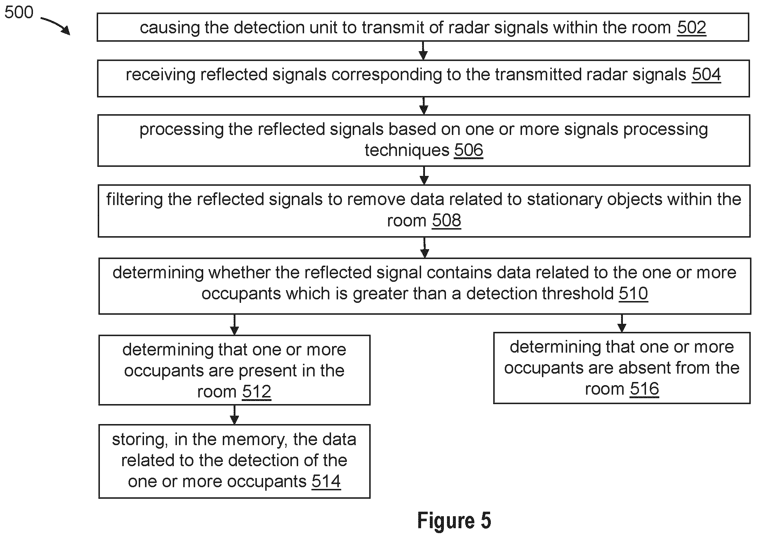

**Figure 5** illustrates a process flow depicting a method 500 for processing the signals from the detection unit 122 to detect the presence of one or more occupants in the room 120. At step 502, the method 500 comprises causing the detection unit 122 to transmit of radar signals within the room 120. At step 504, the method 500 comprises receiving reflected signals corresponding to the transmitted radar signals.

At step 506, the method 500 comprises processing the reflected signals based on one or more signals processing techniques. At step 508, the method 500 comprises filtering the reflected signals to remove data related to stationary objects 128 within the room 120. At step 510, the method 500 comprises determining whether the reflected signal contains data related to the one or more occupants which is greater than a detection threshold. Upon determining that the reflected signals contain data related to the one or more occupants greater than the detection threshold, at step 512, the method 500 comprises determining that one or more occupants are present in the room 120. At step 514, the method 500 comprises storing, in the memory, the data related to the detection of the one or more occupants. In some embodiments, the stored data may be accessed and processed to determine additional data, such as, activity and preferences of the detected one or more occupants. Upon determining that the reflected signals contain data related to the one or more occupants lesser than the detection threshold, at step 516, the method 500 comprises determining that one or more occupants are absent from the room 120. In some embodiments, the method 500 may be repeated at pre-determined time intervals.

While the above steps of Figures 3-5 are shown and described in a particular sequence, the steps may occur in variations to the sequence in accordance with various embodiments of the disclosure. Further, a detailed description related to the various steps of Figures 3-5 is already covered in the description related to Figures 1-2 and is omitted herein for the sake of brevity.

In some embodiments, the one or more processors 202 may be configured to execute instructions included in a computer program product. The computer program product may be embodied in a non-transitory computer readable medium. The computer program product may comprise instructions that, when executed by the one or more processors 202, cause the one or more processors 202 to perform the method as depicted in Figures 3-5.

The disclosure provides methods and systems for accurately monitoring occupancy in rooms. Dual Impulse UWB radars are used to cover all blind spots and cover all areas within the rooms. Occupant related parameters can be accurately extracted and room occupancy is accurately reported. The system may be integrated in a thermostat, leading to simple installation. Further, details related to vital signs of occupants may be determined, and in case of health risks, timely action can be taken. Further, maintenance costs are reduced as predictive alerts may be generated. Furthermore, operational costs are reduced as time of housekeeping and administrative staff is reduced.

In addition, a database may be created that includes information related to preferences of the occupants and the usage of the room by the occupants. Information related to the occupants may be collected, such as, rest timings, sleep quality, preferred room areas, activity maps, and the like. Accordingly, automated control of components within the room can be carried out based on occupant preferences. Further, information related to usage of the room may be utilized to restructure the rooms, leads to better infrastructure and marketing opportunities.

As would be apparent to a person in the art, various working modifications may be made to the methods disclosed herein in order to implement the inventive concept as taught herein.

Moreover, the actions of any flow diagram need not be implemented in the order shown; nor do all of the acts necessarily need to be performed. Also, those acts that are not dependent on other acts may be performed in parallel with the other acts.

The drawings and the forgoing description give examples of embodiments. Those skilled in the art will appreciate that one or more of the described elements may well be combined into a single functional element. Alternatively, certain elements may be split into multiple functional elements. Elements from one embodiment may be added to another embodiment. For example, orders of processes described herein may be changed and are not limited to the manner described herein.

Benefits, other advantages, and solutions to problems have been described above with regard to specific embodiments. However, the benefits, advantages, solutions to problems, and any component(s) that may cause any benefit, advantage, or solution to occur or become more pronounced are not to be construed as a critical, required, or essential feature or component of any or all the claims.

While specific language has been used to describe the subject matter, any limitations arising on account thereto, are not intended. As would be apparent to a person in the art, various working modifications may be made to the method in order to implement the inventive concept as taught herein. The drawings and the foregoing description give examples of embodiments. Those skilled in the art will appreciate that one or more of the described elements may well be combined into a single functional element. Alternatively, certain elements may be split into multiple functional elements. Elements from one embodiment may be added to another embodiment.

## Claims

1. A system for monitoring occupancy in a room, the system comprising:
a detection unit, and
a control unit communicatively connected with the detection unit, the control unit comprising one or more processors configured to:
cause, via the detection unit, transmission of radar signals within the room over a predefined period of time;
receive, via the detection unit, reflected signals corresponding to the transmitted radar signals, the reflected signals being indicative of information associated with the room;
determine, based on the reflected signals, presence of one or more occupants within the room; and
in response to determining presence of the one or more occupants within the room, determine one or more of:
an activity associated with the one or more occupants within the room,
preferences associated with usage of the room by the one or more occupants within the room, and
a visualization map associated with the room.

2. The system of claim 1, wherein the detection unit comprises a first ultra-wide band (UWB) radar and a second UWB radar, wherein the first UWB radar is configured to transmit radar signals within the room, and wherein the second UWB radar is configured to transmit the radar signals through wall structures within the room, thereby transmitting the radar signals throughout the room.

3. The system of claim 1 or 2, wherein the information associated with the room comprises one or more of:
occupant activity parameters related to activities of the one or more occupants within the room,
occupant location parameters related to respective location of the one or more occupants within the room, and
occupant vital parameters indicative of an identification of the one or more occupants within the room and vital sign information associated with the one or more occupants within the room.

4. The system of claim 1, 2 or 3, wherein to determine presence of the one or more occupants, the one or more processors are configured to:
compare one or more of frequency values, phase values, and amplitude values of the transmitted radar signals with one or more of frequency values, phase values, and amplitude values associated with the received reflected signals, respectively;
detect a change in the one or more of frequency values, phase values, and amplitude values of the reflected signals compared to the transmitted radar signals; and
in response to detecting the change, determine presence of the one or more occupants within the room.

5. The system of any preceding claim, wherein to determine the activity associated with the one or more occupants within the room, the one or more processors are configured to:
extract the occupant activity parameters based on the reflected signals; and
determine an activity of the one or more occupants within the room based on the occupant activity parameters, wherein
the activity of the one or more occupants comprises one or more of sleeping, standing, sitting, exercising, and walking.

6. The system of claim 3, wherein the one or more processors are configured to:
extract the occupant vital parameters based on the reflected signals;
determine, based on the occupant vital parameters, a health status associated with the one or more occupants within the room, wherein the health status comprises information related to sleep quality, sleep time, sleep stage, stress level, and health risks associated with the one or more occupants; and
cause the health status to be displayed on a user interface associated with one or more of: the control unit, a user device of the one or more occupants, and a user device of one or more administrators associated with the room; optionally
wherein the one or more processors are configured to:
upon determining the health status to comprise information related to health risks, transmit an alert notification to one or more of a user device of the one or more occupants, and the user device of one or more administrators associated with the room.

7. The system of claim 3 or claims 4 to 6 when dependent on claim 3, wherein to determine the preferences associated with the one or more occupants within the room, the one or more processors are configured to:
extract the occupant activity parameters and the occupant location parameters based on the reflected signals;
detect one or more surrounding parameters associated with the room, the one or more surrounding parameters being associated with a status of surroundings of the one or more occupants; and
determine the preferences associated with a usage of the room based on the occupant activity parameters, the occupant location parameters, and the one or more surrounding parameters; optionally
wherein the one or more processors are configured to:
perform a control action based on the preferences associated with the one or more occupants, wherein the control action comprises adjusting a status of surroundings of the occupant, wherein the status of surroundings comprises one or more of temperature status, light status, usage of one or more room areas, and stationary object status within the room.

8. The system of any preceding claim, wherein to determine the visualization map associated with the room, the one or more processors are configured to:
extract, based on reflected signals, the information associated with the room;
generate a virtual model of the room based on a pre-stored configuration of the room and the information associated with the room, the virtual model comprising a representation of the occupant within the room; and
display the virtual model on one or more of a user interface associated with the one or more occupants and a user interface of an administrator associated with the room.

9. A method for monitoring occupancy in a room, the method comprising:
causing, via a detection unit, transmission of radar signals within the room over a predefined period of time;
receiving, via the detection unit, reflected signals corresponding to the transmitted radar signals, the reflected signals being indicative of information associated with the room;
determining, based on the reflected signals, presence of one or more occupants within the room; and
in response to determining presence of the one or more occupants within the room, determining one or more of:
an activity associated with the one or more occupants within the room,
preferences associated with usage of the room by the one or more occupants within the room, and
a visualization map associated with the room.

10. The method of claim 9, wherein the detection unit comprises a first ultra-wide band (UWB) radar and a second UWB radar, wherein the first UWB radar is configured to transmit radar signals within the room, and wherein the second UWB radar is configured to transmit the radar signals through wall structures within the room, thereby transmitting the radar signals throughout the room.

11. The method of claim 9 or 10, wherein the information associated with the room comprises one or more of:
occupant activity parameters related to activities of the one or more occupants within the room,
occupant location parameters related to respective location of the one or more occupants within the room, and
occupant vital parameters indicative of an identification of the one or more occupants within the room and vital sign information associated with the one or more occupants within the room.

12. The method of claim 9, 10 or 11, wherein determining presence of the one or more occupants within the room comprises:
comparing one or more of frequency values, phase values, and amplitude values of the transmitted radar signals with one or more of frequency values, phase values, and amplitude values associated with the received reflected signals, respectively;
detecting a change in one or more of the frequency values, phase values, and amplitude values of the reflected signals compared to the transmitted radar signals; and
in response to detecting the, determining presence of the one or more occupants within the room; and/or
wherein determining the activity associated with the one or more occupants within the room comprises:
extracting the occupant activity parameters based on the reflected signals; and
determining an activity of the one or more occupants within the room based on the occupant activity parameters, wherein
the activity of the one or more occupants comprises one or more of sleeping, standing, sitting, exercising, and walking.

13. The method of claim 11, further comprising:
extracting the occupant vital parameters based on the reflected signals;
determining, based on the occupant vital parameters, a health status associated with the one or more occupants within the room, wherein the health status comprises information related to sleep quality, sleep time, sleep stage, stress level, and health risks associated with the one or more occupants; and
causing the health status to be displayed on a user interface associated with one or more of: the control unit, a user device of the one or more occupants, and a user device of one or more administrators associated with the room; optionally
further comprising:
upon determining the health status to comprise information related to health risks, transmitting an alert notification to one or more of a user device of the one or more occupants, and the user device of one or more administrators associated with the room.

14. The method of claim 11, wherein determining the preferences associated with the one or more occupants within the room comprises:
extracting the occupant activity parameters and the occupant location parameters based on the reflected signals,
detecting one or more surrounding parameters associated with the room, the one or more surrounding parameters being associated with a status of surroundings of the one or more occupants; and
determining the preferences associated with a usage of the room based on the occupant activity parameters, the occupant location parameters, and the one or more surrounding parameters; optionally
further comprising:
performing a control action based on the preferences associated with the one or more occupants, wherein the control action comprises adjusting a status of surroundings of the occupant, wherein the status of surroundings comprises one or more of temperature status, light status, usage of one or more room areas, and stationary object status within the room.

15. The method of any of claims 9 to 14, wherein determining the visualization map associated with the room comprises:
extracting, based on the reflected signals, the information associated with the room;
generating a virtual model of the room based on a pre-stored configuration of the room and the information associated with the room, the virtual model comprising a representation of the occupant within the room; and
displaying the virtual model on one or more of a user interface associated with the one or more occupants and a user interface of an administrator associated with the room.
